# EUROPEAN PATENT SPECIFICATION

(11) **EP 0 588 177 B1**
(45) Date of publication and mention of the grant of the patent: **26.01.2000**
(21) Application number: 93114084.2
(22) Date of filing: 02.09.1993
(51) Int. Cl.: C12N 15/12, C07K 1/22, C07K 14/715, C12P 21/02, C12P 21/08, C12N 15/62, C12N 1/21, C12N 5/10, A61K 38/17, C12N 5/20

(54) **An interferon-alpha/beta binding protein, its preparation and pharmaceutical compositions containing it**
Interferon-Alpha/Beta bindendes Protein, seine Herstellung und seine enthaltende pharmazeutische Zusammensetzungen
Protéine se liant à l'interferon-alpha/beta, sa préparation et les compositions pharmaceutiques la contenant

(30) Priority: 03.09.1992 IL 103052; 04.08.1993 IL 106591
(43) Date of publication of application: 23.03.1994
(73) Proprietor: YEDA RESEARCH AND DEVELOPMENT CO., Ltd., Rehovot 76 110 (IL)
(72) Inventor: Novick, Daniela, Rehovot (IL); Rubinstein, Menachem, Givat Shmuel (IL)
(74) Representative: VOSSIUS & PARTNER

(56) References cited:
- EP-A- 0 369 877
- FR-A- 2 657 881
- JOURNAL OF CHROMATOGRAPHY vol. 510 , 27 June 1990 , AMSTERDAM NL pages 331 - 337 NOVICK D;ENGELMANN H;WALLACH D;LEITNER O;REVEL M;RUBINSTEIN M; 'Purification of soluble cytokine receptors from normal human urine by ligand-affinity and immunoaffinity chromatography.'
- JOURNAL OF BIOLOGICAL CHEMISTRY. vol. 267, no. 4 , 5 February 1992 , BALTIMORE US pages 2802 - 2809 LUTFALLA G;GARDINER K;PROUDHON D;VIELH E;UZE G; 'The structure of the human interferon alpha/beta receptor gene.'
- FEBS LETTERS. vol. 314, no. 3 , 21 December 1992 , AMSTERDAM NL pages 445 - 448 NOVICK D;COHEN B;RUBINSTEIN M; 'Soluble interferon-alpha receptor molecules are present in body fluids.'
- : "", EXPERIENTIA, , , vol. 45, no. , pages 500 to 508
- : "", EXPERIENTIA, , , vol. 45, no. , pages 508 to 513
- COLAMONICI ET AL.: "", J. BIOL. CHEM., , , vol. 270, no. 14, pages 8188 to 8193
- COLAMONICI ET AL.: "", J. BIOL. CHEM., , , vol. 269, no. 13, pages 9598 to 9602

## Description

The present invention relates to an interferon-α/β binding protein (IFN-α/β binding protein), its muteins and fused proteins and the salts, functional derivatives and active fractions thereof, as well as to its production and pharmaceutical compositions containing it.

Type I interferons (interferon-α and interferon-β, (IFN-α and IFN-β)) constitute a family of structurally related cytokines, defined by their ability to confer resistance to viral infections. Many other biological activities of type I IFNs have been reported, including inhibition of cell proliferation, induction of class I MHC antigens and several other immunoregulatory activities⁽¹⁾. IFN-α and IFN-β are useful for the treatment of several viral diseases including hepatitis-C^{(2,3)} and viral warts^{(4,5)} as well as certain malignancies such as hairy cell leukemia⁽⁶⁾, chronic myelogeous leukemia⁽⁷⁾ and Kaposi's sarcoma⁽⁸⁾.

As in the case of other cytokines, IFN-α exerts its biological activities by binding to a cell surface receptor, which is specific for all IFN-α subtypes, as well as for IFN-β⁽⁹⁾. A human IFN-α receptor was identified and cloned from Daudi cells⁽¹⁰⁾. The cloned receptor has a single transmembrane domain, an extracellular and an intracellular domain. When expressed in murine cells, this receptor confers them responsive to human IFN-αB, but not significantly to other IFN-α and IFN-β species, indicating that additional components may be involved in the response to IFN-β and to various IFN-α subtypes.

Several other studies indicate that there are additional components or receptor subunits involved in the binding of IFN-α and IFN-β⁽¹¹⁻¹³⁾. Furthermore, it was claimed that the already described receptor⁽¹⁰⁾ is involved in binding of all IFN-α and IFN-β species⁽¹⁴⁾.

Soluble cytokine receptors which correspond to the extracellular ligand binding domains of the respective cell associated receptors have been identified in the past. These include among others the soluble receptors of IL-6, IFN-gamma^{(15,16,17)}, TNF⁽¹⁸⁾, IL-1^{(19,20)}, IL-4⁽¹⁹⁾ and IL-2⁽²¹⁾.

The present invention provides an IFN-α/β binding protein including the internal amino acid sequence Met-Val-Lys-Phe-Pro-Ser-Ile-Val-Glu-Glu-Glu-Leu-Gln-Phe-Asp-Leu-Ser-Leu-Val-Ile-Glu-Glu-Gln, a fusion protein containing said protein, muteins, functional derivatives or active fractions, or salts of any one of the foregoing which still bind IFN-α/β.

The previously described receptor⁽¹⁰⁾ is herein referred to as "IFN-αRA" and its soluble analog is herein referred to as "sIFN-αRA".

The IFN-α/β binding protein of the present invention may be produced from natural sources, i.e. it may be derived from human fluids such as sera obtained from healthy or sick people, or from urine.

The IFN-α/β binding protein of the present invention may also be produced by recombinant DNA methods.

The present invention thus also provides methods for the preparation of the IFN-α/β binding protein.

One such method comprises isolation of the binding protein from human fluids by passing the fluid through column to which IFN-α or IFN-β is coupled, and elution of the bound IFN-α/β binding protein. The binding protein is then purified to homogeneity by a size exclusion chromatographic step.

Another method comprises isolation of the binding protein from human fluids by passing the fluid through a column to which anti-IFN-α/β receptor antibody is coupled and elution of the bound binding protein.

Preferably the human fluids are urine or human serum.

In contrast to the soluble form of the known IFN-αRA, the IFN-α/β binding protein inhibits the biological activities of human IFN-α2, IFN-αB, IFN-αC and IFN-β. Protein sequence analysis of IFN-α/β binding protein reveals that its sequence is different from any other known protein, including IFN-αRA.

In view of these findings, it is possible that the IFN-α/β binding protein according to the invention may be an IFN-α/β receptor component, or a soluble form of a novel IFN-α/β receptor.

This invention further concerns DNA molecules comprising the nucleotide sequence encoding the above disclosed IFN-α/β binding protein, a fusion protein containing said protein, muteins, functional derivatives or active fractions thereof, replicable expression vehicles containing said DNA molecules, hosts transformed therewith and protein produced by expression of such transformed hosts. The term "DNA molecules" includes genomic DNA, cDNA, synthetic DNA and combinations thereof.

The invention also relates to DNA molecules which hybridize to the above DNA molecules and encode proteins which still bind IFN-α/β.

The invention also relates to pharmaceutical compositions comprising the above disclosed IFN-α/β binding protein.

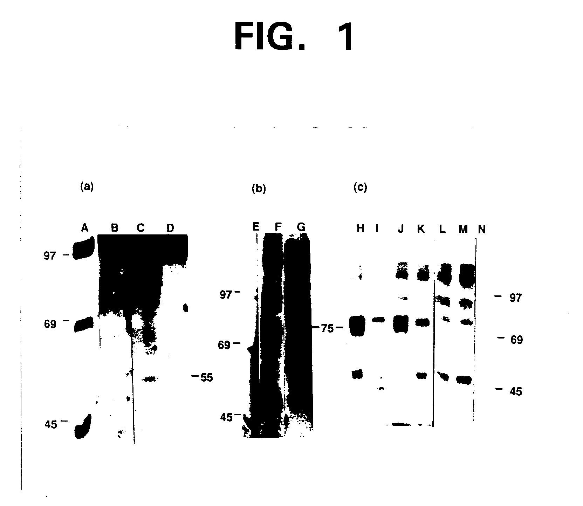

Figure 1 shows:
(a) Western blotting of human sera with anti-IFN-αRA Mab (No. 21.4). Lanes:- A: molecular weight markers (K); B: normal human serum (NHS, 5 µl); C and D: serum from a hairy cell leukemia (HCL) patient (5 and 1 µl respectively).
(b) Autoradiogram of cross-linked complexes consisting of ¹²⁵I-labeled-IFN-αB (apparent molecular weight 25K) and an IFN-α binding protein in body fluids. Lanes:- E: molecular weight markers (K); F: serum (5 µl) from HCL patient cross-linked to ¹²⁵I-IFN-αB; G: NHS (5 µl) cross-linked to ¹²⁵I-IFN-αB.
(c) Autoradiogram of the cross-linked complexes of (b), following immunoprecipitation (Iptn) with anti-IFN-α MAb No. 74-3⁽²²⁾. Lanes: H-K: serum (50 µl) from HCL patient cross-linked to ¹²⁵I-IFN-αB in the absence (lanes H and J) or in the presence of an excess of cold IFN-α2 (Lane I) or excess of cold IFN-αB (Lane K). Lanes L and M: normal human serum (50 µl) cross-linked to ¹²⁵I-IFN-αB in the absence (lane L) or in the presence (lane M) of excess cold IFN-α2; Lane N: molecular weight markers (K).

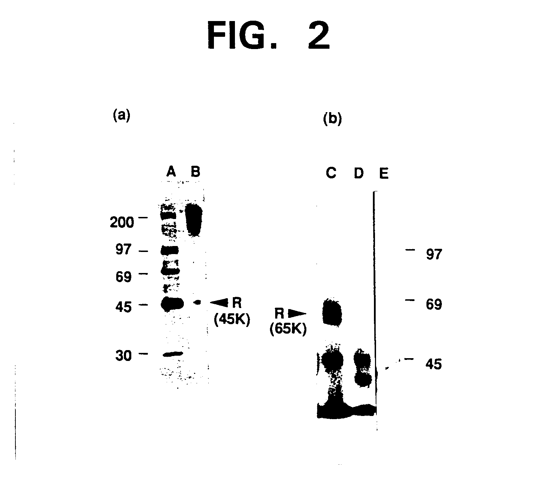

Figure 2 shows:
(a) Western blotting of sIFN-αRA from urine with anti-IFN-αRA Mab No. 21.4. Lanes: A: molecular weight markers (K); B: crude urine (1 µl)
(b) Autoradiogram of a cross-linked complex of urinary IFN-α binding protein and ¹²⁵I-IFN-αB following Iptn. Lanes:- C: urinary proteins, 100-fold concentrated (100 µl) cross-linked to ¹²⁵I-IFN-αB and immunoprecipitated with anti-IFN-α MAb No. 74-3⁽²²⁾; D: same as C except that excess unlabeled IFN-αB was added prior to cross-linking; E: molecular weight markers (K).

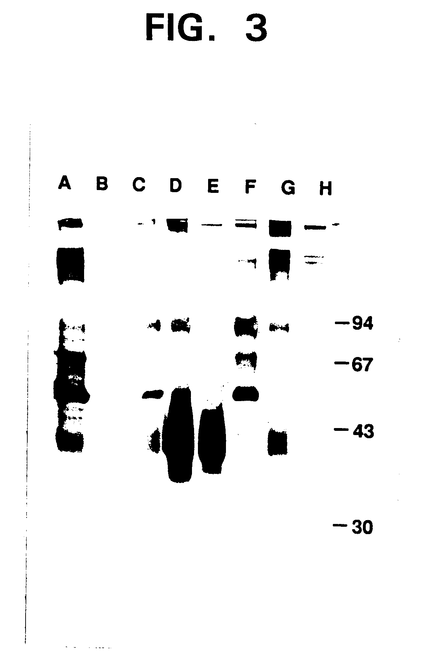

Figure 3 shows SDS-PAGE (sodium dodecyl sulfate polyacrylamide gel electrophoresis) of ligand affinity purified IFN-α/β binding protein. Crude urinary proteins (10 g, from 250 L urine) were loaded on either IFN-α2-agarose or IFN-β-agarose, the columns were washed and bound proteins eluted at pH 2.2. Eluted fractions were neutralized and aliquots were analyzed by SDS-PAGE (10%) under non-reducing conditions and silver staining. The lanes are: A: crude urinary proteins (1.5 µg, load); B-E: final column wash (B) and elutions from the IFN-α2-agarose column:- C: elution 1 (90 ng); D: elution 2 (600 ng); E: elution 3 (150 ng); F-H: elutions from the IFN-β column:- F: elution 1 (450 ng); G: elution 2 (120 ng); H: elution 3 (45 ng). Molecular weight markers (K) are indicated on the right side.

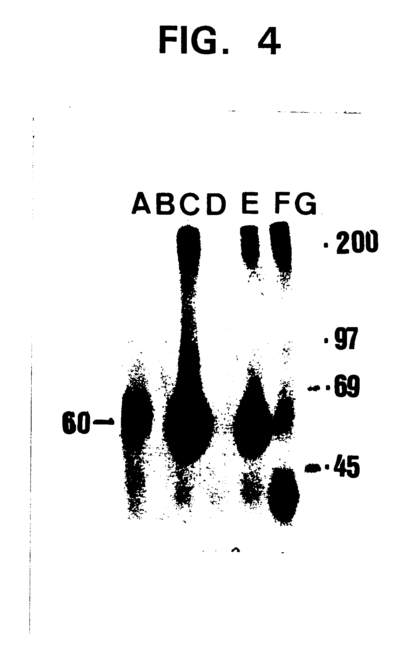

Figure 4 shows an autoradiogram of cross-linked complexes with ¹²⁵I-IFn-α2 (apparent molecular weight 20K) following iptn with anti-IFN-α flab No. 74-3 and SDS-PAGE (7.5%). Lanes: A, B: crude urinary proteins (100 µl) cross-linked in the absence (A) or presence(B) of excess unlabeled IFN-α2; C,D: elution 2 (50 µl) of the IFN-α2-agarose column in the absence (C) or presence (D) of excess unlabeled IFN-α2; E,F: elution 2 (50 µl) of the IFN-β-agarose column in the absence (E) or presence (F) of excess unlabeled IFN-α2; G: Molecular weight markers (indicated on the right side) in K.

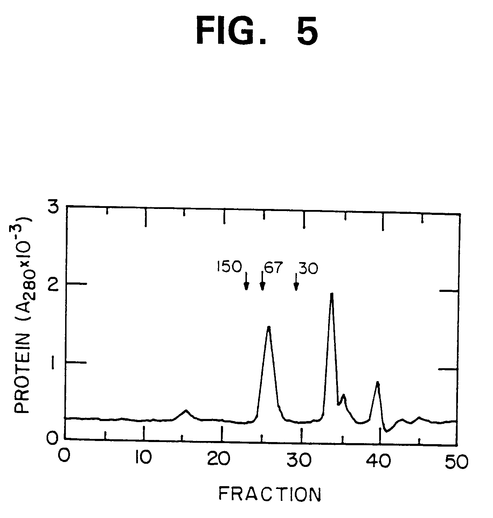

Figure 5 shows size exclusion chromatography of urinary IFN-α/β binding protein from the IFN-α-agarose column (elution 2, 300µl) performed on a superose 12 column (1 x 30cm, Pharmacia, Sweden) pre-equilibrated in phosphate-buffered saline (PBS) at a flow rate of 0.5 ml/min. Fractions of 1 min. (0.5ml) were collected. The column was monitored by UV at 280 nm. The column was calibrated with the indicated molcular weight markers (in K): 30 - carbonic anhydrase; 67 - bovine serum albumin; and 150 - immunoglobulin.

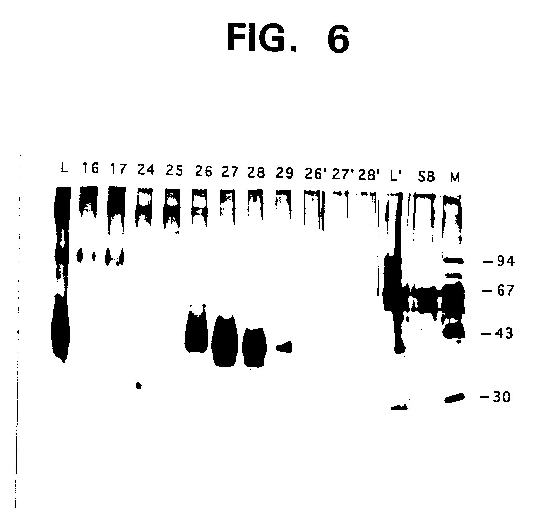

Figure 6 shows SDS-PAGE (10%) under non-reducing conditions of aliquots of purified IFN-α/β binding protein. The gel was stained with silver. The lanes are: Load IFN-α/β binding protein purified on IFN-α-agarose; lanes 16, 17, 24-29: fractions (30µl) 16, 17, and 24-28 respectively, of the superose 12 column of Fig. 5; lanes 26'-28': fractions 26-28 respectively, of a superose 12 column of IFN-α/β binding protein, purified with IFN-β-agarose; lane L': IFN-α/β binding protein purified on IFN-β-agarose; lane SB: the sample buffer; and lane M: molecular weight markers, as indicated on the right side.

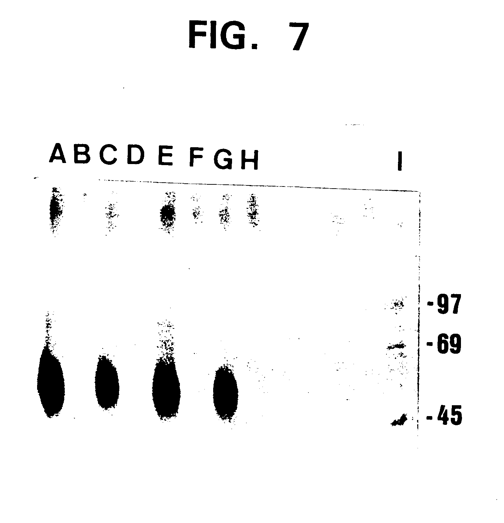

Figure 7 shows an autoradiogram of cross-linked complexes following iptn with anti-IFN-α Mab No. 74-3⁽²²⁾. Lanes: A and B aliquot of urinary IFN-α/β binding protein, partially purified on IFN-α2-agarose and cross-linked to ¹²⁵I-IFN-α2 (10⁶ cpm) in the absence, (lane A) or presence (lane B) of excess unlabeled IFN-α2. Lanes C and D: the same procedure as in lanes A, B, carried out with aliquots of homogenous IFN-α/β binding protein from fraction 26 of the superose 12 column (see Figure 5). Lanes E, F and G,H - the same procedure as with lanes C, D, but with fractions 27 and 28 (respectively) of the superose 12 column. Molecular weight markers (K) are indicated on the right side.

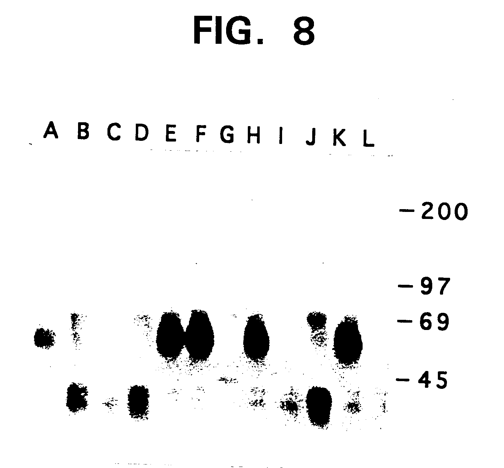

Figure 8 shows an autoradiogram of cross-linked complexes of homogenous IFN-α/β binding protein with ¹²⁵I-IFN-α2 in the presence or absence of competing IFNs. The lanes are: A-D cross-linking of IFN-α/β binding protein from the IFN-α2-agarose in the presence of excess unlabeled IFN-αC (3-fold, lane A); IFN-αB (33-fold, lane B); IFN-α2 (670-fold, lane C); IFN-β (400-fold, lane D); lane E: cross-linking in the absence of competing IFN; lane F: cross-linking in the presence of excess 400-fold unlabeled IFN-gamma; lane G: molecular weight markers (indicated on the right side); lanes H-K: cross-linking of IFN-α/β binding protein from the IFN-β-agarose column in the absence (lane H) or presence of excess unlabeled IFN-α2 (670-fold, lane I), IFN-β (400-fold, lane J) and IFN-gamma (400-fold, lane K); lane L: ¹²⁵I-IFN-α2 cross-linked to itself (no receptor).

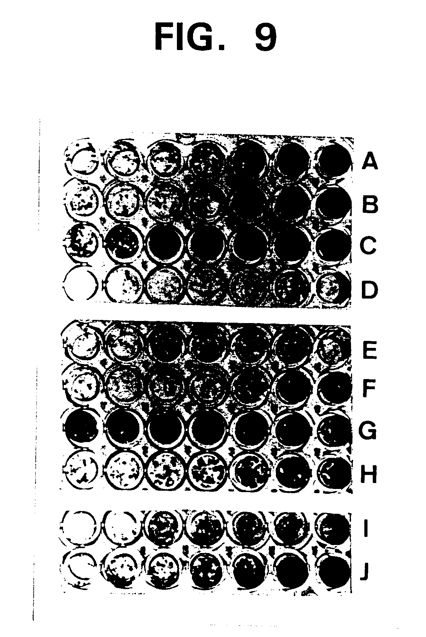

Figure 9 shows the inhibition of the antiviral activity of IFN by IFN-α/β binding protein. IFN activity is titrated by twofold dilutions from right to left. Protected cells appear dark in the well, whereas virus- lysed cells appear light. The rows are as follows:
A. Standard (std) IFN-β (20 u/ml in the right-most well), diluted twofold.
B. Std IFN-β as above, plus neutralizing anti-IFN-α Mab No. 9-3⁽²²⁾.
C. Titration of leaked IFN-α2 present in fraction 27 of IFN-α/β binding protein, purified by IFN-α2-agarose and superose 12 columns.
D. Neutralization of the leaked IFN-α2 antiviral activity in fraction 27 of the superose 12 column with Mab 9-3.
E. Std IFN-β (20 u/ml in the right-most well) diluted twofold, plus anti-IFN-α Mab 9-3 plus IFN-α/β binding protein (fraction 27, 40 ng/well), showing complete and specific inhibition of IFN-β by IFN-α/β binding protein from the IFN-α2-agarose column.
F. Std IFN-β (20 u/ml in the right-most well) diluted twofold.
G. Std IFN-gamma (10 u/ml in every well) plus IFN-α/β binding protein from IFN-α2 column (fraction 27, 40 ng) plus Mab anti-IFN-α 9-3.
H. Std. IFN-gamma (20 u/ml in the right-most well) diluted twofold, plus Mab anti-α2 9-3.
I. Std IFN-α2 (20 u/ml in the right-most well) diluted twofold, plus polyclonal anti-IFN-β antibodies, plus IFN-α/β binding protein (fraction 27, ∼0.4 ng/well) from the IFN-β agarose/superose 12 columns, showing complete and specific inhibition of IFN-α2 by IFN-α/β binding protein from the IFN-β-agarose column.
J. Std IFN-α2 dilutions as in I plus polyclonal anti-IFN-β antibodies.

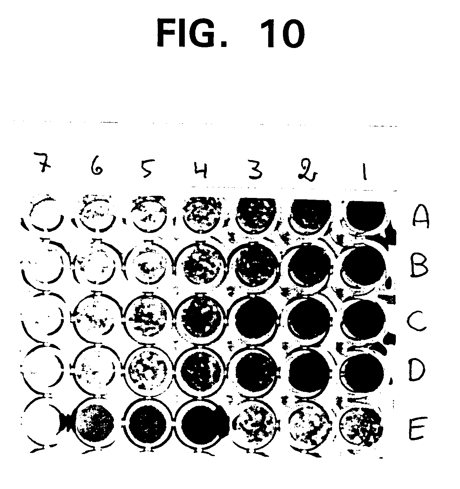

Figure 10 shows the inhibition of the antiviral activity of IFN-αB and IFN-αC by IFN-α/β binding protein. (For details, see Fig. 9).

The rows are:
A. IFN-αB (20 u/ml in the right-most well);
B. Same as A together with anti-IFN-α2 Mab No. 9-3, showing that the Mab does not neutralize IFN-αB;
C: IFN-αC (20 u/ml in the right-most well);
D: Same as C, together with anti-IFN-α2 Mab No. 9-3, showing that the Mab does not neutralize IFN-αC;
   - Well E1:: IFN-αB (20 u/ml) plus Mab No. 9-3, plus IFN-α/β binding protein (fraction 27, from IFN-α2 agarose/superose 12 columns, 0.1 µg) showing complete inhibition of IFN-αB by IFN-α/β binding protein.
   - Well E2:: IFN-αC (20 u/ml) plus Mab No. 9-3, plus IFN-α/β binding protein (fraction 27, from IFN-α2 agarose/superose 12 columns, 0.1 µg) showing complete inhibition of IFN-αC by IFN-α/β binding protein.
   - Well E3:: IFN-β (20 u/ml) plus Mab No. 9-3, plus IFN-α/β binding protein (fraction 27 from IFN-α2 agarose/superose 12 columns, 0.1 µg) showing complete inhibition of IFN-β by IFN-α/β binding protein.
   - Wells E4-E6:: Control IFN-αB, IFN-αC and IFN-β, respectively, (20 u/ml each) in the presence of Mab No. 9-3.
   - Well E7:: Control virus only.

### DETAILED DESCRIPTION OF THE INVENTION

Soluble forms of the known⁽¹⁰⁾ IFN-α receptor were identified in human serum and urine with the aid of anti-IFN-αRA monoclonal antibodies. Detectable levels of sIFN-αRA were found in sera of hairy cell leukemia patients that were undergoing IFN-α treatment, while the level of sIFN-αRA in normal human serum was below the limit of detection. This indicates that IFN treatment may increase shedding or release of sIFN-αRA⁽²³⁾. A similar increase of the 50K IFN-α binding protein was observed in serum of IFN-α treated patients as compared with the serum of healthy controls.

The soluble IFN-αRA found in serum is a protein of molecular weight 55K as was determined by Western blotting with monoclonal antibody 21.4, directed against recombinant IFN-αRA. Using the same techniques, we obtained indications that a soluble IFN-αRA of similar size is also present in culture medium of human Daudi cells. The results obtained with normal urine indicate that it contains a sIFN-αRA having a molecular weight which is lower by about 10K than the molecular weight of the soluble receptor from serum and cell culture medium.

In a parallel set of experiments, serum (Fig. 1c) and urine (Fig. 2b) were cross-linked to ¹²⁵I-IFN-αB (molecular weight 25K) and immunoprecipitated with Mab anti-IFN-α No. 74-3. Cross-linked products of an IFN-α binding protein molecular weights of about 75K (serum) and about 65K (urine) were identified. These bands were initially assumed to be products of the soluble version of the already known IFN-αRA⁽¹⁰⁾, however, upon isolation and analysis (see below) it was found that another protein, IFN-α/β binding protein is present in serum and urine.

The IFN-α/β binding protein was then isolated from normal urine by a procedure that included two chromatographic steps. Crude urinary proteins were loaded on a column consisting of IFN-α2 bound to agarose. The column was washed and bound proteins were eluted at low pH. Eluted proteins were then resolved by size exclusion HPLC to give several protein peaks, one of which, eluting in fractions 26, 27, 28 (Fig. 5), was identified by its ability to react specifically with ¹²⁵I-IFN-α2. This protein was further characterized by N-terminal microsequence analysis which gave the following major sequence, at its N-terminal domain: 
Asp-Ser-Pro-Asp-Tyr-Thr-Asp-Glu-Ser-Arg-Thr-Phe-Lys-Ile-Arg-Leu-Arg
1 . . . 5 . . . . 10 . . . . 15 . .
 In addition, a polypeptide having three additional amino acid residues (ILE-XXX-Tyr) at the N-terminus of the major sequence was detected as a minor component (xxx denotes an unidentified amino acid, however it is assumed that this amino acid is Cys, which cannot be detected by the method employed. A lesser possibility is that this acid is Ser). The resulting sequence is completely different from the one of the known IFN-αRA⁽¹⁰⁾. It is also different from that of any other known protein and it is not coded by any known DNA sequence as determined by comparing it to Swissprot and Genebank data libraries, using both FastA and TFastA programs⁽²⁴⁾. Hence this is a novel protein, referred to as IFN-α/β binding protein. This pure IFN-α/β binding protein did not react with Mab 21.4 as determined by Western blotting and immunoprecipitation indicating again that it is different from the known IFN-αRA.

A sample of the IFN-α/β binding protein was digested with CNBr, resolved on SDS-PAGE and one of the resulting digestion fragments which was a peptide smaller than 10 K gave the following internal sequence (Met precedes the actual sequence): 
Met-Val-Lys-Phe-Pro-Ser-Ile-Val-Glu-Glu-Glu-Leu-Gln-Phe-Asp-Leu-
 .   .   .   .   5   .   .   .   .  10   .   .   .   .  15   .
Ser-Leu-Val-Ile-Glu-Glu-Gln
 .   .   .  20   .   .  23
 The binding protein was also isolated from normal urine by chromatography on a column consisting of IFN-β bound to agarose, followed by size exclusion chromatography. Analysis of fractions 26-29 from the IFN-α agarose/superose 12 procedure and fractions 26-28 from the IFN-β agarose/superose 12 procedure by SDS-PAGE and staining with silver gave the same single band of molecular weight 40,000 (Fig. 6, lanes 26-29 vs 26'-28', respectively). Hence the combination of ligand affinity chromatography on either IFN-α or IFN-β agarose followed by size exclusion chromatography yielded in both cases a homogenous IFN-α/β binding protein. Since the previously described receptor⁽¹⁰⁾ does not react well with IFN-β, it is obvious that the newly isolated IFN-α/β binding protein differs from the known receptor in its binding properties.

The binding of ¹²⁵I-IFN-α2 to IFN-α/β binding protein, isolated either on the IFN-α2 agarose or the IFN-β agarose column was inhibited by various type I IFNs, including IFN-α2, IFN-αB, IFN-αC and IFN-β, but not by (type II) IFN-gamma.

The homogenous urinary IFN-α/β binding protein retained the ability to bind its labeled ligand (¹²⁵I-IFN-α2), and following covalent cross-linking, a complex of molecular weight 60K was formed. The molecular weight corresponded to a 1:1 complex of IFN-α/β binding protein and the 20K IFN-α2. An excess of unlabeled IFN-α2 added to the cross-linking reactions abolished the signal, thereby proving the specificity of the interaction between this protein and IFN-α (Fig. 7).

Furthermore, the pure IFN-α/β binding protein inhibited the antiviral activity of IFN-α2, IFN-αB, IFN-αC and IFN-β and not IFN-gamma; which indicates that it is a general type I IFN binding protein. As such it is a useful agent, capable of blocking or modulating the biological activities of IFN-α and IFN-β. Since homogenous IFN-α/β binding protein binds type I IFNs (IFN-α and IFN-β), we conclude that such binding does not require association with any other polypeptide. Hence, IFN-α/β binding protein seems to function as an autonomous IFN-α/β binding protein and is not necessarily a subunit of a more complex receptor, which e.g., comprises both IFN-αRA and the IFN-α/β receptor.

So far the physiological role of the soluble cytokine receptors has not been established. Two mechanisms of formation of these receptors have been proposed: proteolytic cleavage of the membrane anchored receptor, e.g., the soluble IL-2R ⁽²⁵⁾ and alternative splicing of mRNA as in the case of IL-4 ⁽²⁶⁾ and IL-7 ⁽²⁷⁾. The soluble receptors bind their specific ligands and modulate their activity either by inhibiting the biological activity, as was shown in the TNF system ^{(28,29)}, or by enhancing their activity as demonstrated with the IL-6 system ⁽³⁰⁾. The recombinant soluble TNF receptor (also known as TBP-TNF binding protein) was found to prevent septic shock in animal models ⁽³¹⁾ and soluble forms of IL-1 receptor were found to have profound inhibitory effects on the development of in vivo alloreactivity in mouse allograft recipients⁽³²⁾.

Similarly the IFN-α/β binding protein of the present invention may find use as a modulator of IFN-α and IFN-β activity, e.g. in type I diabetes⁽³³⁾ and in autoimmune diseases, in graft rejections, in AIDS and in similar diseases, in which there is an aberrant expression of IFN-α⁽³⁴⁾ or IFN-β. It may thus be used in any condition where an excess of IFN-α or IFN-β is endogenously produced, or exogenously administered.

The IFN-α/β binding protein and its muteins, fused proteins and their salts, functional derivatives, and active fractions thereof are indicated for the treatment of autoimmune diseases and other inflammations in mammals.

The present invention further relates to pharmaceutical compositions comprising a pharmaceutically acceptable carrier and the IFN-α/β binding protein of the invention or its active muteins, fused proteins and their salts, functional derivatives or active fractions thereof.

The method of administration can be via any of the accepted mode of administration for similar agents and will depend on the condition to be treated, e.g. intravenously, intramuscularly, subcutaneously, by local injection or topical application, or continuously by infusion, etc.

The pharmaceutical compositions of the invention are prepared for administration by mixing the IFN-α/β binding protein or its derivatives, with physiologically acceptable carriers, stabilizers and excipients, and prepared in dosage form, e.g., by lyophilization in dosage vials. The amount of active compound to be administered will depend on the route of administration, the disease to be treated and the condition of the patient. Local injection, for instance, will require a lower amount of the protein on a body weight basis than will intravenous infusion.

As used herein the term "muteins" refers to analogs of the IFN-α/β binding protein in which one or more of the amino acid residues of the natural IFN-α/β binding protein are replaced by different amino acid residues or are deleted, or one or more amino acid residues are added to the natural sequence of the IFN-α/β binding protein, without changing considerably the activity of the resulting product. These muteins are prepared by known synthesis and/or by site-directed mutagenesis techniques, or any other known technique suitable therefor.

The term "fused protein" refers to a polypeptide comprising the IFN-α/β binding protein or a mutein thereof fused with another protein which has an extended residence time in body fluids. The IFN-α/β binding protein may thus be fused to another protein, polypeptide or the like, e.g., an immunoglobulin or a fragment thereof.

The term "salts" herein refers to both salts of carboxyl groups and to acid addition salts of amino groups of the IFN-α/β binding protein, muteins and fused proteins thereof. Salts of a carboxyl group may be formed by means known in the art and include inorganic salts, for example, sodium, calcium, ammonium, ferric or zinc salts, and the like, and salts with organic bases as those formed, for example, with amines, such as triethanolamine, arginine or lysine, piperidine, procaine and the like. Acid addition salts include, for example, salts with mineral acids such as, for example, hydrochloric acid or sulfuric acid, and salts with organic acids such as, for example, acetic acid or oxalic acid.

"Functional derivatives" as used herein cover derivatives of the IFN-α/β binding protein and its fused proteins and muteins, which may be prepared from the functional groups which occur as side chains on the residues or the N- or C-terminal groups, by means known in the art, and are included in the invention as long as they remain pharmaceutically acceptable, i.e. they do not destroy the activity of the protein and do not confer toxic properties on compositions containing it. These derivatives may, for example, include polyethylene glycol side-chains which may mask antigenic sites and extend the residence of the IFN-α/β binding protein in body fluids. Other derivatives include aliphatic esters of the carboxyl groups, amides of the carboxyl groups by reaction with ammonia or with primary or secondary amines, N-acyl derivatives of free amino groups of the amino acid residues formed with acyl moieties (e.g. alkanoyl or carbocyclic aroyl groups) or O-acyl derivatives of free hydroxyl groups (for example that of seryl or threonyl residues) formed with acyl moieties.

As "active fractions" of the IFN-α/β binding protein, its fused proteins and its muteins, the present invention covers any fragment or precursors of the polypeptide chain of the protein molecule alone or together with associated molecules or residues linked thereto, e.g., sugar or phosphate residues, or aggregates of the protein molecule or the sugar residues by themselves, provided said fraction has the same biological and/or pharmaceutical activity.

The invention will now be illustrated by the following non-limiting examples:

### Materials

Sera of patients were obtained from Dr. Dan Aderka (Ichilov Hospital, Tel-Aviv, Israel). Crude urinary proteins of normal individuals, concentrated 1000-fold⁽¹⁵⁾, were obtained from Serono Laboratories, Rome, Italy; immobilized anti-IFN-α MAb No. 74.3 on agarose hydrazide (Sigma) was prepared as described before⁽²²⁾; di-N-hydroxy-succinimidyl suberate (DSS) was from Pierce.

### Interferons

Recombinant IFN-α2 (2X10⁸ units/mg) was kindly provided by Dr. C. Weismann, University of Zurich. IFN-α2 was labeled by a modification of the Chloramine T method ⁽³⁵⁾. Briefly, 7µg of IFN-α was labeled with 1 mCi of Na¹²⁵I in the presence of 1 mg/ml of chloramine T (20 sec. on ice), to a specific activity of 4X10⁷ cpm/µg. IFN-αB was from Ciba-Geigy; IFN-αC and IFN-β were from Inter-Lab Ltd., Ness-Ziona, Israel.

### EXAMPLE 1: Western blotting of human sera and urine with Mab 21.4

Samples of normal human serum (NHS), hairy cell leukemia serum (HCL) and crude urinary proteins, were subjected to SDS-PAGE under non-reducing conditions⁽³⁶⁾ and electroblotted (in 25 mM Tris, 1.92 mM glycine, 20% methanol) onto nitrocellulose sheets (Schleicher and Schuell, 0.45 µm). Following electroblotting the sheet was incubated with a Blocking Buffer (10% non-fat milk in PBS containing 0.05% Tween-20 and 0.02% sodium azide) and then for 2 hrs at room temperature with the anti-IFN-αRA antibody 21.4, prepared against recombinant IFN-αRA. The nitrocellulose sheet was washed with 0.05% Tween-20 in PBS and incubated overnight at 4°C with ¹²⁵I-goat anti-mouse antibodies (0.7X10⁶ cpm/ml, in the Blocking Buffer). The blot was then washed, dried and autoradiographed.

The HCL serum exhibited a weak band of molecular weight 55K (Figure 1, lanes C and D), while no such band was observed in NHS (Figure 1, lane B). The high molecular weight protein seen in lanes B-D (ca. 150K) was most probably immunoglobulin.

A sample of crude urinary proteins (1 µl 1000-fold concentrated) was subjected to SDS-PAGE followed by Western blotting with anti-IFN-αRA MAb 21.4. A protein band of molecular weight 45K was detected (Fig. 2a). The high molecular weight protein seen in the same lane (150K) was identified by protein sequence analysis as human immunoglobulin that could be detected in 1000-fold concentrated urine due to cross-reaction of the second antibody (^{¹²⁵}I-goat anti-mouse antibodies) with the human immunoglobulins.

### EXAMPLE 2: Cross-linking and Immunoprecipitation of crude serum and urine

Samples of serum or urine were incubated (1 hr at 4°C) with ¹²⁵I-IFN-αB (300,000 cpm) in the absence or in the presence of a 100-fold excess of unlabeled IFN-α2. DSS, dissolved in dimethyl sulfoxide (Me₂SO), was then added to a final concentration of 1 mM and the mixture was left for 20 min. at 4°C. The reaction was stopped by the addition of 1M Tris-HCl pH 7.5, and 1M NaCl to a final concentration of 100 mM. The samples were immunoprecipitated by the addition of anti-IFN-α MAb 74-3, immobilized on agarose hydrazide (25 µl, 7 mg/ml) ⁽²²⁾. Following incubation (overnight at 4°C), the beads were washed 3 times with PBS, suspended in a sample buffer containing 2% mercaptoethanol and the supernatants were analysed by SDS-PAGE followed by autoradiography.

A specific but weak band of molecular weight 75K could be observed in HCL serum even without immunoprecipitation (Figure 1, lane F). The cross-linked product was enriched by Iptn with immobilized anti-IFN-α MAb 74-3. Indeed a broad band centering around 75K, probably consisting of a 50K protein cross-linked to the 25 K ¹²⁵I-IFN-αB, was clearly observed in the HCL sera (lane H and J) but not in the NHS (lane L). The specificity of this binding was verified by repeating the cross-linking experiments but in the presence of an excess of cold IFN-αB and IFN-α2. Indeed the 75K band was significantly reduced (lanes I and K). In addition to the specific (displacable) 75K band, some non-displacable bands (50, 80, 97 and >100K) were observed. The 80K band (clearly seen in lanes H-M) could not be completely resolved from the 75K band.

The ligand binding capacity of the urinary 40K protein was checked by incubating a sample of crude urine with ¹²⁵I-IFN-αB in the presence or absence of excess of unlabeled IFN-αB, and then cross-linking with DSS. Following Iptn with anti-IFN-α MAb 74-3 and SDS-PAGE, a specific cross-linked product of molecular weight 65K was detected (Figure 2b). This complex was smaller by 10K than the one obtained from serum.

### EXAMPLE 3: Isolation of an IFN-α/β binding protein

IFN-α2 (5 mg) was coupled to Affigel-10 (1ml, BioRad), according to the manufacturer's instructions and packed into a column. Crude urinary proteins (1000-fold concentrated, 10 g, 250 ml) were loaded onto the column at a flow rate of 0.25 ml/min. The column was washed with 250ml 0.5M NaCl in phosphate buffered saline (PBS) followed by PBS (10 ml). Bound proteins were then eluted (75 µg) with 0.25mM citric acid, pH 2.2, immediately neutralized by 1M Na₂CO₃. 1ml fractions were collected. The fractions were analyzed by SDS-PAGE and silver staining (Fig. 3), and the protein content was determined with fluorescamine. The various fractions were analyzed by cross-linking with ¹²⁵I-IFN-α2, iptn, SDS-PAGE and autoradiography as described in Example 2. An IFN-α/β binding protein was found in crude urine and in fractions eluted from both the IFN-α2-agarose and the IFN-β-agarose columns (Fig. 4).

Eluted fractions were applied to a superose 12 column (1 X 30cm, Pharmacia, Sweden), in aliquots (0.3 ml, 11 µg). The column was pre-equilibrated and eluted with phosphate buffered saline and sodium azide (0.02%) at a flow rate of 0.5 ml/min and fractions of 1 min. were collected (Fig. 5). The IFN-α/β binding protein eluted in fractions 26-28 (7 µg) as a homogenous 40K protein, as determined by SDS-PAGE and silver staining (Fig. 6). About 25 micrograms of pure protein were recovered from 250 l. of urine. Similar results were obtained when crude urinary proteins were purified on an IFN-β-agarose column followed by a superose 12 column.

### EXAMPLE 4: Protein sequence analysis

Fraction 27 (4 microgram protein) of the Superose 12 column of Example 3 was adsorbed on a PVDF membrane (Pro-Spin, Applied Biosystems, USA) and the membrane was subjected to protein sequence analysis on a Model 475 microsequencer (Applied Biosystems, USA).

The following major sequence was obtained: 
Asp-Ser-Pro-Asp-Tyr-Thr-Asp-Glu-Ser-Arg-Thr-Phe-Lys-Ile-Arg-Leu-Arg
1 . . . 5 . . . . 10 . . . . 15 . .
 In addition, a secondary polypeptide having three additional amino acid residues (Ile-xxx-Tyr) at the N-terminus of the major sequence was detected (xxx denotes an unidentified amino acid, however it is assumed that this amino acid is Cys, which cannot be detected by the method employed. A lesser possibility is that this acid is Ser).

The resulting sequence is completely different from that of the already known IFN-α receptor A⁽¹⁰⁾ and is different from any other known protein. It is also different from any protein coded by a known DNA sequence as determined by searching Swissprot and Genebank databases by the programs FastA and TFastA⁽²⁴⁾. Hence this protein is a novel protein.

A sample of the IFN-α/β binding protein was digested with CNBr, resolved on SDS-PAGE and blotted onto a PVDF membrane. One of the peptides, smaller than 10 K, gave the following internal sequence (Met precedes the actual sequence): 
Met-Val-Lys-Phe-Pro-Ser-Ile-Val-Glu-Glu-Glu-Leu-Gln-Phe-Asp-Leu-
 .   .   .   .   5   .   .   .   .  10   .   .   .   .  15   .
Ser-Leu-Val-Ile-Glu-Glu-Gln
 .   .   .  20   .   .  23
 Since the pure sIFN-α receptor did not contain a sequence corresponding to IFN-αRA, we conclude that the urinary binding protein described in example 2 is not sIFN-αRA. It is possible that the major sIFN-α receptor in serum is also the IFN-α/β binding protein, and not sIFN-αRA.

### EXAMPLE 5: Cross-linking and immunoprecipitation of pure IFN-α/β binding protein

Samples of urinary IFN-α/β binding protein, purified on IFN-α2 or IFN-β columns, or further purified on the Superose 12 column, were incubated (1 hr at 4°C) with ¹²⁵I-IFN-α2 (10⁶ cpm) in the absence or in the presence of a 100-fold excess of unlabeled IFN-α2. DSS dissolved in dimethyl sulfoxide (Me₂SO) was then added to a final concentration of 1 mM and the mixture was left for 20 min. at 4°C. The reaction was stopped by the addition of 1 M Tris-HCl pH 7.5, and 1 M NaCl to a final concentration of 100 mM. The samples were immunoprecipitated by the addition of anti-IFN-α MAb 74-3 immobilized on agarose hydrazide (25 µl, 7 mg/ml)⁽²²⁾. Following incubation (overnight at 4°C), the beads were washed twice with PBS, suspended in a sample buffer containing 2% mercaptoethanol and the supernatants were analysed by SDS-PAGE (7.5% acrylamide) followed by autoradiography. A specific cross-linked product of molecular weight of about 60K was seen in all fractions and was completely eliminated in the presence of excess unlabeled IFN-α2 (Fig. 7).

### EXAMPLE 6: Displacement of ¹²⁵I-IFN-α2 by various type I IFNs in the cross-linking experiment

Homogeneous IFN-α/β binding protein (40 ng) was incubated (1 hr at 4°C) with ¹²⁵I-IFN-α (10⁶ cpm) in the absence or in the presence of a 3-670- fold excess of unlabeled IFN-α2, IFN-αB, IFN-αC, IFN-β or IFN-gamma. DSS dissolved in dimethyl sulfoxide (Me₂SO) was then added to a final concentration of 1 mM and the mixture was left for 20 min. at 4°C. The reaction was stopped by the addition of 1 M Tris-HCl pH 7.5, and 1 M NaCl to a final concentration of 100 mM. The samples were immunoprecipitated by the addition of anti-IFN-α MAb 74-3 immobilized on agarose hydrazide (25 µl, 7 mg/ml)⁽²²⁾. Following incubation (overnight at 4°C), the beads were washed twice with PBS, suspended in a sample buffer containing 2% mercaptoethanol and the supernatants were analysed by SDS-PAGE (7.5% acrylamide) followed by autoradiography (Fig. 6). It was found that all type I IFNs were equally effective in inhibiting the binding of ¹²⁵I-IFN-α2 to IFN-α/β binding protein. This result indicates that IFN-α/β binding protein is different from the previously described IFN-αRA, which is specific to IFN-αB only⁽¹⁰⁾.

### EXAMPLE 7: Inhibition of IFN-α and IFN-β activity by IFN-α/β binding protein

The experiment was based on the cytopathic effect (CPE) inhibition assay which is used for measuring IFN activity⁽³⁷⁾. The antiviral activity was calibrated against NIH reference standards of human IFN-α, IFN-β and IFN-gamma. The following procedure was used:
Confluent monolayers of WISH cells (ATCC CCL-25) were prepared on day 1, seeding 20,000 cells/well in 100µl of MEM supplemented with 10% FCS, in 96-flat bottom well plates. The plates were incubated at 37°C in 5% CO₂. On day 2, standard IFN-β (10µl, 1000 U/ml) was diluted twofold serially in a separate plate (65 µl). Soluble IFN-α/βR (10 µl, 4 µg/ml, purified on IFN-α column and superose 12 column) was added to the standard IFN-β dilutions. Neutralizing monoclonal anti-IFN-α antibody (25 µl, No. 9-3⁽²²⁾) sufficient for neutralizing 1000 U/ml of IFN-α, was added to each well in order to neutralize IFN-α2 that leaked from the IFN-α2 agarose column. The mixture was left for 90 min. at 37°C and then transferred to the plate with the WISH cells, followed immediately by challenge with vesicular stomatitis virus (VSV, 50 µl, 220 PFU/cell). The plates were incubated overnight at 37°C. The assay was calibrated against standard IFN-β. On day 3, about 20 hrs following virus challenge, the cytopathic effect was scored microscopically and the monolayer was stained with crystal violet (Fig. 9).

It was found that IFN-α/β binding protein completely inhibited the antiviral action of IFN-β (Fig. 7, E). Based on the extent of inhibition, a minimal titer of 2.5 x 10⁵ anti-IFN-β units/mg of receptor was calculated. In a parallel experiment, IFN-α2 was similarly neutralized by IFN-α/β binding protein that was purified on an IFN-β column. The test was carried out in the presence of neutralizing polyclonal anti-IFN-β antibodies (Fig. 9, I). Another experiment revealed that IFN-α/β binding protein inhibited the antiviral activity of IFN-αB and IFN-αC to the same extent as it inhibited IFN-β (Fig. 10).

### EXAMPLE 8: Immunization of mice, cell fusion and screening

Balb/c mice are injected 4 times with a homogenous preparation of urinary IFN-α/β binding protein (1 µg/mouse/injection). The mouse showing the highest titer by inverted sRIA (see below) is chosen for fusion. Its splenic lymphocytes are fused with an NSO-1 myeloma variant (NSO) cells, kindly provided by C. Milstein, MRC, Cambridge, U.K.). Hybridoma supernatants are tested for the presence of anti-IFN-α/βR antibodies by an inverted sRIA. 96-well plates are coated with affinity purified goat anti-mouse antibodies followed by the addition of hybridoma supernatants and ¹²⁵I-IFN-α/βR. In parallel, hybridomas are checked for neutralizing of the IFN-α/β antiviral activity. Hybridomas that are found to secrete anti-IFN-α/βR antibodies are cloned and recloned by the limiting dilution technique.

### EXAMPLE 9: Recombinant production of IFN-α/β binding protein

In order to produce recombinant IFN-α/β binding protein, the polypeptide sequence is back translated into two oligonucleotide sequences, corresponding to the following peptide sequences: 
1)  Tyr-Asp-Ser-Pro-Asp-Tyr-Thr-Asp-Glu (256 permutations)
2)                          Tyr-Thr-Asp-Glu-Ser-Arg-Thr-Phe-Lys-Ile
                                                    (192 permutations)
 The probes are 5'-labeled with [³²P] and used to screen genomic and cDNA libraries containing the gene and cDNA corresponding to IFN-α/β binding protein mRNA. Positive clones are isolated, their sequence is determined and if they contain a complementary sequence they are used as more specific probes to isolate a cDNA clone corresponding to the full length mRNA.

The isolated cDNA is subjected to site directed mutagenesis with appropriate oligo-nucleotides so that a termination codon and a polyadenylation site are inserted after the last essential codon of the IFN-α/β binding protein. This construct is then inserted into appropriately constructed expression vectors by techniques well known in the art⁽³⁸⁾. Double-stranded cDNA is linked to plasmid vectors by homopolymeric tailing or by restriction linking involving the use of synthetic DNA linkers or blunt-ended ligation techniques. DNA ligases are used to ligate the DNA molecules and undesirable joining is avoided by treatment of DNA strands with alkaline phosphatase.

Alternatively, oligonucleotide mixture corresponding to peptide 1) is mixed together with either oligo(dT) or an oligomer corresponding to one of the two flanking sequences within phage lambda used for constructing the cDNA library. The mixtures are used as primers for a polymerase chain reaction (PCR) with a DNA template from the entire cDNA library. Amplified DNA bands are characterized by sequence analysis to confirm the presence of a region coding for the polypeptide of Example 4. The cDNA comprising the soluble IFN-α/βR mRNA is then introduced into an expression vector and the recombinant protein is produced and purified by means known in the art.

The production of a fused protein comprising the IFN-α/β binding protein and, e.g., the constant region of IgG₁ heavy chain may be carried out as follows: the DNA IFN-α/β binding protein is subjected to site-directed mutagenesis with appropriate oligonucleotides so that a unique restriction site is introduced immediately after the coding sequence. A plasmid bearing the constant region of IgG₁ heavy chain, e.g. pRKCO4₂Fc₁⁽³⁹⁾ is subjected to similar site- directed mutagenesis to introduce the same unique restriction site as close as possible to Asp 216 of IgG₁ heavy chain in a way that allows translation in phase of the fused protein. A dsDNA fragment consisting of 5' untranslated sequences and encoding for IFN-α/β binding protein is prepared by digestion at the unique restriction sites. The mutated pRKCD4₂Fc₁ is similarly digested to generate a large fragment containing the plasmid and the IgG₁ sequences. The two fragments are then ligated to generate a new plasmid encoding a polypeptide precursor consisting of the N-terminal IFN-α/β binding protein and about 227 C-terminal amino acids of IgG₁ heavy chain (hinge region and CH2 and CH3 domains). The DNA encoding the fused protein may be isolated from the plasmid by digestion with appropriate restriction enzymes and then inserted into an efficient expression vector.

In order to be capable of expressing a IFN-α/β binding protein, its muteins or the fused proteins, an expression vector should comprise also specific nucleotide sequences containing transcriptional and translational regulatory information linked to the DNA coding for the desired protein in such a way as to permit gene expression and production of the protein. First, in order for the gene to be transcribed, it must be preceded by a promoter recognizable by RNA polymerase, to which the polymerase binds and thus initiates the transcription process. There are a variety of such promoters in use, which work with different efficiencies (strong and weak promoters). They are different for prokaryotic and eukaryotic cells.

The promoters that can be used in the present invention may be either constitutive, for example, the int promoter of bacteriophage lambda, the bla promoter of the β-lactamase gene of pBR322, and the CAT promoter of the chloramphenicol acetyl transferase gene of pPR325, etc., or inducible, such as the prokaryotic promoters including the major right and left promoters of bacteriophage lambda (P_{L} and P_{R}), the trp, recA, lacZ, lacI, ompF and gal promoters of E. coli, or the trp-lac hybrid promoter, etc. (Glick, B.R. (1987) J. Ind. Microbiol. 1:277-282).

Besides the use of strong promoters to generate large quantities of mRNA, in order to achieve high levels of gene expression in prokaryotic cells, it is necessary to use also ribosome-binding sites to ensure that the mRNA is efficiently translated. One example is the Shine-Dalgarno sequence (SD sequence) appropriately positioned from the initiation codon and complementary to the 3'-terminal sequence of 16S RNA.

For eukaryotic hosts, different transcriptional and translational regulatory sequences may be employed, depending on the nature of the host. They may be derived from viral sources, such as adenovirus, bovine papilloma virus, Simian virus, or the like, where the regulatory signals are associated with a particular gene which has a high level of expression. Examples are the TK promoter of Herpes virus, the SV40 early promoter, the yeast ga14 gene promoter, etc. Transcriptional initiation regulatory signals may be selected which allow for repression and activation, so that expression of the genes can be modulated.

The DNA molecule comprising the nucleotide sequence coding for IFN-α/β binding protein of the invention or its fragments or muteins or fused proteins thereof, and the operably linked transcriptional and translational regulatory signals is inserted into a vector which is capable of integrating the desired gene sequences into the host cell chromosome. In order to be able to select the cells which have stably integrated the introduced DNA into their chromosomes, one or more markers which allow for selection of host cells which contain the expression vector is used. The marker may provide for prototrophy to an auxotropic host, biocide resistance, e.g., antibiotics, or heavy metals, such as copper, or the like. The selectable marker gene can either be directly linked to the DNA gene sequences to be expressed, or introduced into the same cell by cotransfection. Additional elements may also be needed for optimal synthesis of single chain binding protein mRNA. These elements may include splice signals, as well as transcription promoters, enhancers, and termination signals. cDNA expression vectors incorporating such elements include those described by Okayama, H., (1983) Mol.Cel.Biol. 3:280.

In a preferred embodiment, the introduced DNA molecule will be incorporated into a plasmid or viral vector capable of autonomous replication in the recipient host. Factors of importance in selecting a particular plasmid or viral vector include: the ease with which recipient cells that contain the vector may be recognized and selected from those recipient cells which do not contain the vector; the number of copies of the vector which are desired in a particular host; and whether it is desirable to be able to "shuttle" the vector between host cells of different species.

Preferred prokaryotic vectors include plasmids such as those capable of replication in E. coli, for example, pBR322, ColEl, pSC101, pACYC 184, etc. (see Maniatis et al., op.cit.); Bacillus plasmids such as pC194, pC221, pT127, etc. (Gryczan, T., "The Molecular Biology of the Bacilli", Academic Press, NY (1982), pp. 307-329); Streptomyces plasmids including pIJ101 (Kendall, K.J. et al. (1987) J.Bacteriol. 169:4177-4183); Streptomyces bacteriophages such as φC31 (Chater, KF. et al., in "Sixth International Symposium on Actinomycetales Biology", Akademiai Kaido, Budapest, Hungary (1986), pp. 45-54), and Pseudomonas plasmids (John, J.F., et al. (1986) Rev. Infect. Dis. 8:693-704), and Izaki, K. (1978) Jpn. J. Bacteriol. 33:729-742).

Preferred eukaryotic plasmids include BPV, vaccinia, SV40, 2-micron circle, etc., or their derivatives. Such plasmids are well known in the art (Botstein, D., et al. (1982) Miami Wint.Symp. 19:265-274; Broach, JR., in "The Molecular Biology of the Yeast Saccharomyces: Life Cycle and Inheritance", Cold Spring Harbor Laboratory, Cold Spring Harbor, NY, pp. 445-470 (1981); Broach, J.R., (1982) Cell 28:203-204; Bollon, D.P., et al. (1980) J. Clin. Hematol. Oncol. 10:39-48; Maniatis, T., in "Cell Biology: A Comprehensive Treatise, Vol. 3: Gene Expression", Academic Press, NY, pp. 563-608 (1980)).

Once the vector or DNA sequence containing the construct(s) has been prepared for expression, the expression vector may be introduced into an appropriate host cell by any of a variety of suitable means, such as transformation, transfection, lipofection, conjugation, protoplast fusion, electroporation, calcium phosphate precipitation, direct microinjection, etc.

Host cells to be used in this invention may be either prokaryotic or eukaryotic. Preferred prokaryotic hosts include bacteria such as E. coli, Bacillus, Streptomyces, Pseudomonas, Salmonella, Serratia, etc. The most preferred prokaryotic host is E. coli. Bacterial hosts of particular interest include E. coli K12 strain 294 (ATCC 31446), E. coli X1776 (ATCC 31537), E. coli W3110 (F⁻, lambda⁻, prototropic (ATCC 27325)), and other enterobacterium such as Salmonella typhimurium or Serratia narcescens and various Pseudomonas species. Under such conditions, the protein will not be glycosylated. The prokaryotic host must be compatible with the replicon and control sequences in the expression plasmid.

However, since the IFN-α/β binding protein is probably a glycosylated protein, eukaryotic hosts are preferred over prokaryotic hosts. Preferred eukaryotic hosts are mammalian cells, e.g., human, monkey, mouse and chinese hamster ovary (CHO) cells, because they provide post-translational modifications to protein molecules including correct folding, correct disulfide bond formation as well as glycosylation at correct sites. Also yeast cells and insect cells can carry out post-translational peptide modifications including high mannose glycosylation. A number of recombinant DNA strategies exist which utilize strong promoter sequences and high copy number of plasmids which can be utilized for production of the desired proteins in yeast and in insect cells. Yeast cells recognize leader sequences on cloned mammalian gene products and secrete peptides bearing leader sequences.

After the introduction of the vector, the host cells are grown in a selective medium, which selects for the growth of vector-containing cells. Expression of the cloned gene sequence(s) results in the production of the IFN-α/β binding protein, a fusion protein, or a mutein or a fragment thereof. The expressed protein is then isolated and purified by any conventional procedure involving extraction, precipitation, chromatography, electrophoresis, or the like, or by affinity chromatography, using anti-IFN-α/β binding protein monoclonal antibodies immobilized on a gel matrix contained within a column. Crude preparations containing said recombinant IFN-α/β binding protein are passed through the column whereby the IFN-α/β binding protein will be bound to the column by the specific antibody while the impurities will pass through. After washing, the protein is eluted from the gel at a high or a low pH, eg. pH 11 or pH 2.

### References

1. Taylor, J.L. and Grossberg, S.E. (1990), Recent progress in interferon research: molecular mechanisms of regulation, action, and virus circumvention, Virus Research 15, 1-26.
2. Bisceglie A.M., Martin, P., Kassianides, C., Lisker-Melman, M., Murray, L., Waggoner, J., Goodmann, Z., Banks, M.S. and Hoofnagle, J.H. (1989). Recombinant interferon alpha therapy for chronic hepatitis C. A randomized, double-bind, placebo-controlled trail, NEJM 321, 1506-1510.
3. McDonnel, W.M. and Elta G.H. (1992) Acute Hepatitis C infection: Interferon finally succeeds. Gastroenterology (US), 103:1359-60.
4. Friedman-Kien, A.E., Eron, L.J., Conant, M., Growdon, W., Badiak, H., Bradstreet, P.W., Fedorczyk, D., Trout, R. and Plesse, T.F.(1988), Natural Interferon alfa for treatment of Condylomata Acuminata, J. Am. Med. Assn. 259, 533-538.
5. Mains, J. and Handley, J. (1992) Interferon: Current and future Clinical Uses in infectious Disease Practice. Int. J. Std. AIDS, 3:4-9.
6. Berman, E., Heller, G., Kempin, S., Gee, T., Tran, L. and Clarkson, B. (1990), Incidence of response and long term follow up in patients with Hairy Cell Leukemia treated with recombinant interferon alfa-2a, Blood 75, 839-845.
7. Talpaz M., Kantarjian, H.M., McCredie, K.B., Keating, M.J., Trujillo, J. and Gutterman (1987), Clinical Investigation of human alpha interferon in Chronic Myelogenous Leukemia, Blood 69, 1280-1288.
8. De Wit, R., Schattenkerk, J.K.M.E., Boucher, C.A.B., Bakker, P.J.M., Veenhof, K.H.N. and Danner, S.A. (1988), Clinical and virological effects of high-dose recombinant-α in disseminated Aids-related Kaposi's sarcoma, Lancet 2, 1214-1222.
9. Branka, A.A. and Baglioni, C. (1981) Evidence that types I and II Interferons have different receptors. Nature, 294:768-770.
10. Uze G., Lutfalla, G. and Gresser, I. (1990), Genetic transfer of a functional human interferon α receptor into mouse cells: cloning and expression of its cDNA, Cell 60, 225-234.
11. Colamonici, O.R. et al. (1990) Characterization of three monoclonal antibodies that recognize the interferon-α2 receptor. Proc. Natl. Acad. Sci. USA, 87:7230-7234.
12. Platanias, L.C. et al. (1992) Expression of the IFN-α receptor in hairy cell leukemia, Brit. J. Haematology, 82:541-546.
13. Colaminici, O.R. et al. (1993) Identification of a novel subunit of the type I Interferon receptor localized to human chromosome 21, J. Biol. Chem., 268:10895-10899.
14. Benoit, P. et al. (1993) A monoclonal antibody to recombinant human IFN-α receptor inhibits biologic activity of several species of human IFN-α, IFN-β and IFN-omega, J. Immunol. 150:707-716.
15. Novick, D., Engelmann, H., Wallach, D. and Rubinstein, M. (1989), Soluble cytokine receptors are present in normal human urine, J. Exp. Med. 170, 1409-1414.
16. Novick, D., Engelmann, H., Wallach, D., Leitner, O., Revel, M. and Rubinstein, M. (1990), Purification of soluble cytokine-receptors from normal human urine by ligand-affinity and immuno-affinity chromatography, J. Chromatography 510, 331-337.
17. Novick, D., Engelmann, H., Revel, M., Leitner, O. and Rubinstein M. (1991), Monoclonal antibodies to the soluble IL-6 receptor: affinity purification, ELISA, and inhibition of ligand binding, Hydridoma 10, 137-146.
18. Engelmann, H., Novick, D. and Wallach D. (1990), Two tumor-necrosis-factor binding proteins purified from human urine. Evidence for immunological cross reactivity with cell surface tumor-necrosis-factor receptors. J.Biol.Chem. 265, 1531-1536.
19. Maliszewski, C.R. and Fanslow, W.C. (1990), Soluble receptors for IL-1 and IL-4. Biological activity and therapeutic potential. Trends in Biotechnol. 8, 324-329.
20. Eastgate, J.A., Symons, J.A. and Duff G.W. (1990), Identification of an interleukin-1 beta binding protein in human plasma, FEBS LETTERS 260, 213-216.
21. Marcon, L., Fritz, M.E., Kurman, C.C., Jensen, J.C. and Nelson, D.L. (1988), Soluble Tac peptide is present in the urine of normal individuals and at elevated levels in patients with adult T-cell leukemia, Clin. Exp. Immunol. 73, 29-33.
22. Novick, D., Eshhar, Z. and Rubinstein, M. (1982), Monoclonal antibodies to human α-interferon and their use for affinity chromatography, J. Immunol. 129, 2244-2247.
23. Novick, D., Cohen, B. and Rubinstein, M. (1992), Soluble IFN-α receptor molecules are present in Body Fluids, FEBS Letters, 314:445-448.
24. Pearson, W.R. and Lipman, D.G. (1988), Improved tools for biological sequence comparison, Proc. Natl. Acad. Sci. USA, 85:2444-2448.
25. Josimovic-Alasevic, O., Hermann, T. and Diamanstein, T. (1988), Demonstration of two distinct forms of released low-affinity type interleukin-2 receptors, Eur. J. Immunol. 18, 1855-1857.
26. Mosley,B., Beckman, M.P., March, C., J., Idzerda, R.L., Gimpel, S., D., VandenBos, T., Friend, D., Alpert, A., Anderson, D., Jackson, J., Wignall, J.M. Smith C., Gallis, B., Sims, J.E., Urdal, D., Widmer, M.B., Cosman, D. and Pari, L.S. (1989), The murine interleukin-4 receptor: molecular cloning and characterization of secreted and membrane forms. Cell 59, 335-348.
27. Goodwin, R.G., Friend, D., Ziegler, S.F., March. C.J., Namen, A.E. and Park, L.S. (1990), Cloning of the human and murine interleukin-7 receptors: demonstration of a soluble form and homology to a new receptor superfamily, Cell 60, 941-951.
28. Engelmann, H., Aderka, D., Rubinstein, M., Rotman, D. and Wallach, D. (1989), A tumor necrosis factor-binding protein purified to homogeneity from human urine protects cells from tumor necrosis factor toxicity, J. Biol. Chem. 264, 11974-11980.
29. Seckinger, P., Isaaz, S. and Dayer, J.M. (1988), A human inhibitor of tumor necrosis factor alpha, J. Exp. Med. 167, 1511-1516.
30. Novick, D., Shulman, L., Chen, L. and Revel, M. (1992), Enhancement of interleukin 6 cytostatic effect on human breast carcinoma cells by soluble IL-6 receptor from urine and reversion by monoclonal antibodies, Cytokine 4, 6-11.
31. Lesslauer, W., Taduchi, H., Gentz, R., Shlaeger, E.J., Brockhaus. M., Grau, G., Piguet, P.F., Pointaire, P., Vassalli. P. and Loetscher, H. (1991), Bioactivity of recombinant human TNF receptor fragments, J. Cell. Biochem. (Suppl. 15F):115.
32. Fanslow, F.W., Sims, J.E., Sasenfeld, H., Morrisey, P.J., Gillis, S., Dower, S.K. and Widmer, M.B.(1990), Regulation of alloreactivity in vivo by soluble form of the interleukin-1 receptor, Science 248, 739-742.
33. Stewart, T.A. (1993) Induction of type I diabetes by interferon-α in transgenic mice, Science, 260:1942-6.
34. Klippel, J.H., Carrete, S., Preble, D.T., Friedman, R.M. and Grimley P.M. (1985), Serum alpha interferon and lymphocyte inclusions in systemic lupus erythematosus. Annals of the Rheumatic Diseases. 44, 104-108.
35. Hunter, M.W. (1978). Radioimmunoassay, In: The Handbook of Experimental Immunology, D.M. Weir (ed.) Oxford: Blackwell Press, p. 141.
36. Laemmli, U.K. (1970), Cleavage of structural protein during the assembly of head bacteriophage T4, Nature 227, 680-685.
37. Rubinstein S. et al., (1981) J. Virol. 37:755-758.
38. Maniatis et al., Molecular Cloning: A Laboratory Manual, Cold Spring Harbor Laboratory, New York, 1982
39. Byrn R.A. et al., 1990, Nature (London) 344, 667-670

## Claims

1. An IFN-α/β binding protein including the internal amino acid sequence Met-Val-Lys-Phe-Pro-Ser-Ile-Val-Glu-Glu-Glu-Leu-Gln-Phe-Asp-Leu-Ser-Leu-Val-Ile-Glu-Glu-Gln, a fusion protein containing said protein, muteins, functional derivatives or active fractions, or salts of any one of the foregoing which still bind IFN-α/β.

2. The IFN-α/β binding protein according to claim 1, having a molecular weight of about 40 kD.

3. The IFN-α/β binding protein according to claim 1, having a molecular weight of about 50 kD.

4. A process for the preparation of the IFN-α/β binding protein of any one of claims 1 to 3 comprising isolation of the binding protein from human fluids by passing the fluid through a chromatographic column, followed by purification.

5. The process according to claim 4, in which a chromatographic column to which IFN-α2 is coupled is employed.

6. The process according to claim 4, in which a chromatographic column to which IFN-β is coupled is employed.

7. The process according to claim 4, in which a chromatographic column to which anti-IFN-α/β binding protein antibodies are coupled is employed.

8. The process according to any one of claims 4 to 7, wherein the fluid is urine.

9. The process according to claim 8, wherein the urine employed is in crude form.

10. The process according to claim 8, wherein the urine employed is in the form of concentrated urinary proteins.

11. The process according to any one of claims 4 to 7, wherein the fluid is serum.

12. A DNA molecule comprising the nucleotide sequence encoding the IFN-α/β binding protein of any one of claims 1 to 3, a fusion protein containing said protein, muteins, functional derivatives or active fractions thereof.

13. A DNA molecule which hybridizes to the DNA molecule according to claim 12 and encodes a protein which still binds IFN-α/β.

14. A replicable expression vehicle comprising a DNA molecule according to claim 12 or 13 and capable, in a transformant host cell, of expressing an IFN-α/β binding protein, or a protein having the same biological activity.

15. A host cell transformed with an expression vehicle according to claim 14.

16. A prokaryotic host cell according to claim 15.

17. A eukaryotic host cell according to claim 15.

18. A process for the production of a recombinant soluble IFN-α/β receptor protein, comprising culturing a transformed host cell according to claim 15, and recovering the IFN-α/β binding protein.

19. The IFN-α/β biding protein according to of any one of claims 1 to 3 characterized in that it is a recombinant protein.

20. A pharmaceutical composition comprising the IFN-α/β binding protein, mutein, functional derivative or active fraction or salt thereof of any one of claims 1 to 3 or 19 or the IFN-α/β binding protein produced according to the process of claim 18.

21. The pharmaceutical composition of claim 20 for the treatment of autoimmune diseases and other inflammations in mammals.

22. An antibody directed to the IFN-α/β binding protein according to of any one of claims 1 to 3 or 19 or the IFN-α/β binding protein produced according to the process of claim 18.

23. The antibody according to claim 22, which is a monoclonal antibody.

24. The antibody according to claim 22, which is a polyclonal antibody.

25. A hybridoma producing an antibody according to claim 23.

## Patentansprüche

1. IFN-α/β-bindendes Protein, das die interne Aminosäuresequenz Met-Val-Lys-Phe-Pro-Ser-Ile-Val-Glu-Glu-Glu-Leu-Gln-Phe-Asp-Leu-Ser-Leu-Val-Ile-Glu-Glu-Gln einschließt, ein Fusionsprotein, das das Protein enthält, Muteine, funktionelle Derivate oder aktive Fraktionen, oder Salze von einem der vorgenannten, die noch IFN-α/β binden.

2. IFN-α/β-bindendes Protein nach Anspruch 1, das ein Molekulargewicht von etwa 40 kD besitzt.

3. IFN-α/β-bindendes Protein nach Anspruch 1, das ein Molekulargewicht von etwa 50 kD besitzt.

4. Verfahren zur Herstellung von IFN-α/β-bindendem Protein nach einem der Ansprüche 1 bis 3, umfassend die Isolierung des bindenden Proteins aus menschlichen Flüssigkeiten durch Durchleiten der Flüssigkeit durch eine Chromatographiesäule, gefolgt von einer Aufreinigung.

5. Verfahren nach Anspruch 4, wobei eine Chromatographiesäule verwendet wird, an die IFN-α2 gekoppelt ist.

6. Verfahren nach Anspruch 4, wobei eine Chromatographiesäule verwendet wird, an die IFN-β gekoppelt ist.

7. Verfahren nach Anspruch 4, wobei eine Chromatographiesäule verwendet wird, an die Anti-IFN-α/β-bindendes Protein-Antikörper gekoppelt sind.

8. Verfahren nach einem der Ansprüche 4 bis 7, wobei die Flüssigkeit Urin ist.

9. Verfahren nach Anspruch 8, wobei der Urin in der Rohform verwendet wird.

10. Verfahren nach Anspruch 8, wobei der Urin in der Form von konzentrierten Urinproteinen verwendet wird.

11. Verfahren nach einem der Ansprüche 4 bis 7, wobei die Flüssigkeit Serum ist.

12. DNA-Molekül, umfassend die Nucleotidsequenz, die das IFN-α/β-bindende Protein nach einem der Ansprüche 1 bis 3, ein Fusionsprotein, das das Protein enthält, Muteine, funktionelle Derivate oder aktive Fraktionen davon codiert.

13. DNA-Molekül, das mit dem DNA-Molekül nach Anspruch 12 hybridisiert und ein Protein codiert, das noch IFN-α/β bindet.

14. Replizierbares Expressionsvehikel, das ein DNA-Molekül nach Anspruch 12 oder 13 umfaßt und in der Lage ist, in einer transformierten Wirtszelle ein IFN-α/β-bindendes Protein oder ein Protein, das die gleiche biologische Aktivität besitzt, zu exprimieren.

15. Wirtszelle, die mit einem Expressionsvehikel nach Anspruch 14 transformiert ist.

16. Prokaryontische Wirtszelle nach Anspruch 15.

17. Eukaryontische Wirtszelle nach Anspruch 15.

18. Verfahren zur Herstellung von rekombinantem, löslichem IFN-α/β-Rezeptorprotein, umfassend die Züchtung einer transformierten Wirtszelle nach Anspruch 15 und Gewinnung des IFN-α/β-bindenden Proteins.

19. IFN-α/β-bindendes Protein nach einem der Ansprüche 1 bis 3, dadurch gekennzeichnet, daß es ein rekombinantes Protein ist.

20. Arzneimittel, umfassend das IFN-α/β-bindende Protein, Mutein, funktionelle Derivat oder die aktive Fraktion oder das Salz davon nach einem der Ansprüche 1 bis 3 oder 19 oder das gemäß dem Verfahren nach Anspruch 18 hergestellte IFN-α/β-bindende Protein.

21. Arzneimittel nach Anspruch 20 zur Behandlung von Autoimmunkrankheiten und anderen Entzündungen in Säugern.

22. Antikörper, gerichtet auf das IFN-α/β-bindende Protein nach einem der Ansprüche 1 bis 3 oder 19 oder das gemäß dem Verfahren nach Anspruch 18 hergestellte IFN-α/β-bindende Protein.

23. Antikörper nach Anspruch 22, der ein monoclonaler Antikörper ist.

24. Antikörper nach Anspruch 22, der ein polyclonaler Antikörper ist.

25. Hybridom, das einen Antikörper nach Anspruch 23 produziert.

## Revendications

1. Protéine se liant à l'interféron-α/β comprenant la séquence interne d'acides aminés Met-Val-Lys-Phe-Pro-Ser-Ile-Val-Glu-Glu-Glu-Leu-Gln-Phe-Asp-Leu-Ser-Leu-Val-Ile-Glu-Glu-Gln, une protéine de fusion comprenant ladite protéine, des mutéines, des dérivés fonctionnels ou des fractions actives, ou les sels de n'importe quel élément susmentionné qui lient toujours l'interféron-α/β.

2. Protéine se liant à l'interféron-α/β selon la revendication 1, ayant une masse moléculaire d'environ 40 KD.

3. Protéine se liant à l'interféron-α/β selon la revendication 1, ayant une masse moléculaire d'environ 50 kD.

4. Procédé de préparation de la protéine se liant à l'interféron-α/β selon l'une quelconque des revendications 1 à 3 comprenant l'isolement de la protéine de liaison des fluides humains en faisant passer ces fluides dans une colonne chromatographique, puis par purification.

5. Procédé selon la revendication 4, dans lequel on utilise une colonne chromatographique à laquelle est couplé l'IFN-α2.

6. Procédé selon la revendication 4, dans lequel on utilise une colonne chromatographique à laquelle est couplé l'IFN-β.

7. Procédé selon la revendication 4, dans lequel on utilise une colonne chromatographique à laquelle sont couplés des anticorps de la protéine de liaison anti-IFN-α/β.

8. Procédé selon l'une quelconque des revendications 4 à 7, dans lequel le fluide est de l'urine.

9. Procédé selon la revendication 8, dans lequel l'urine est employée sous sa forme brute.

10. Procédé selon la revendication 8, dans lequel l'urine employée se présente sous la forme de protéines urinaires concentrées.

11. Procédé selon l'une quelconque des revendications 4 à 7, dans lequel le fluide est du sérum.

12. Molécule d'ADN comprenant la séquence de nucléotides codant pour la protéine se liant à l'IFN-α/β selon l'une quelconque des revendications 1 à 3, une protéine de fusion contenant ladite protéine, des mutéines, des dérivés fonctionnels ou des fractions actives de celle-ci.

13. Molécule d'ADN qui s'hybride avec la molécule d'ADN selon la revendication 12 et qui code pour une protéine qui lie toujours l'IFN-α/β.

14. Véhicule d'expression réplicable comprenant une molécule d'ADN selon les revendications 12 ou 13 et capable, dans une cellule hôte transformante, d'exprimer une protéine se liant à l'IFN-α/β, ou une protéine ayant la même activité biologique.

15. Cellule hôte transformée avec un véhicule d'expression selon la revendication 14.

16. Cellule hôte procaryote selon la revendication 15.

17. Cellule hôte eucaryote selon la revendication 15.

18. Procédé pour la production d'un récepteur protéique soluble recombinant pour l'IFN-α/β, comprenant la culture d'une cellule hôte transformée selon la revendication 15 et la récupération de la protéine se liant à l'IFN-α/β.

19. Protéine se liant à l'IFN-α/β selon l'une quelconque des revendications 1 à 3, caractérisée en ce qu'elle est une protéine recombinante.

20. Composition pharmaceutique comprenant la protéine se liant à l'IFN-α/β, la mutéine, le dérivé fonctionnel ou la fraction active, ou le sel correspondant selon l'une quelconque des revendications 1 à 3 ou 19 ou la protéine se liant à l'IFN-α/β produite selon le procédé de la revendication 18.

21. Composition pharmaceutique selon la revendication 20 pour le traitement de maladies auto-immunes ou d'autres inflammations chez les mammifères.

22. Anticorps adressé à la protéine se liant à l'IFN-α/β selon l'une quelconque des revendications 1 à 3 ou 19 ou la protéine se liant à l'IFN-α/β produite selon le procédé de la revendication 18.

23. Anticorps selon la revendication 22, qui est un anticorps monoclonal.

24. Anticorps selon la revendication 22, qui est un anticorps polyclonal.

25. Hybridome produisant un anticorps selon la revendication 23.
